# EUROPÄISCHE PATENTSCHRIFT

(11) **EP 2 384 785 B1**
(45) Veröffentlichungstag und Bekanntmachung des Hinweises auf die Patenterteilung: **23.07.2014**
(21) Anmeldenummer: 11164968.7
(22) Anmeldetag: 05.05.2011
(51) Int. Cl.: A61N 1/05, A61B 5/0478

(54) **Verfahren zum Herstellen einer Elektrodenstruktur sowie Elektrodenstruktur für eine neuronale Schnittstelle**
Method for producing an electrode structure and electrode structure for a neuronal interface
Procédé de fabrication d'une structure d'électrodes et structure d'électrodes pour une interface neuronale

(30) Priorität: 05.05.2010 DE 102010019597
(43) Veröffentlichungstag der Anmeldung: 09.11.2011
(73) Patentinhaber: CorTec GmbH, 79110 Freiburg (DE)
(72) Erfinder: Henle, Christian, 79100, Freiburg (DE); Stieglitz, Thomas, 79110, Freiburg (DE)
(74) Vertreter: Schneider, Günther Martin

(56) Entgegenhaltungen:
- WO-A1-2009/053333
- DE-T2-602005 006 019
- US-A1- 2007 027 514
- US-A1- 2010 023 102

## Beschreibung

### Gebiet der Erfindung

Die Erfindung betrifft ein Verfahren zum Herstellen einer Elektrodenstruktur für eine neuronale Schnittstelle sowie eine Elektrodenstruktur für eine neuronale Schnittstelle, insbesondere zur Implantation in ein Gehirn.

### Hintergrund der Erfindung

Neuronale Schnittstellen, welche in ein Gehirn implantiert werden können, weisen Elektroden auf, welche zur Ableitung von Neuronenaktivitäten, d.h. zum Messen von Gehirnströmen oder zur Stimulation bestimmter Hirnareale dienen. Hierfür werden etwa Elektrokortikogramm-Elektroden (ECoG-Elektroden) oder Cuff Elektroden verwendet.

Die Implantation von ECoG-Elektroden in ein Gehirn ist beispielsweise bei der prächirurgischen Epilepsie-Diagnostik bekannt. ECoG-Elektroden werden auch bei der Resektion von Gehirntumoren eingesetzt, um durch Stimulation bestimmter Gehirnareale zu erkennen, ob es sich bei dem Gehirnareal um ein funktionell wichtiges Gehirnareal handelt, welches auf keinen Fall operativ entfernt werden sollte.

Die mit Hilfe der ECoG-Elektroden gemessenen Gehirnströme können etwa einer Gehirn-Computer-Schnittstelle (Brain-Computer-Interface, BCI) zugeführt und dort ausgewertet werden. Mit dem Ergebnis der Auswertung kann beispielsweise eine Prothese gesteuert werden. Mit Hilfe solcher Gehim-Computer-Schnittstellen können etwa behinderte Menschen mittels ihrer Gedanken Prothesen oder dergleichen steuern.

Mit Cuff-Elektroden können etwa periphere Nerven stimuliert und/oder deren Signale abgeleitet und einer Gehirn-Computer-Schnittstelle zugeführt werden. Cuff Elektroden werden auch in klinischen Anwendungen eingesetzt.

Es ist bekannt, Elektroden für neuronale Schnittstellen sandwichartig zwischen zwei Trägermaterialien, etwa zwischen zwei Silikonfolien, einzubetten.

Um eine möglichst präzise Aufzeichnung von Gehirnströmen bzw. eine möglichst präzise Stimulation bestimmter Hirnareale zu erreichen, ist es notwendig, Elektrodenstrukturen mit einer möglichst hohen Auflösung zu verwenden, wobei die Auflösung durch die Anzahl der Elektrodenkontaktflächen auf einer vorbestimmten Fläche definiert ist. Die Auflösung der Elektrodenstruktur kann dadurch erhöht werden, dass die Anzahl der zwischen den beiden Silikonfolien eingebetteten Elektroden bzw. Elektrodenkontaktflächen erhöht wird.

Die maximale Anzahl von Elektrodenkontaktflächen und damit die maximale Auflösung der Elektrodenstruktur auf dem Trägermaterial sind allerdings begrenzt, weil auf der zur Verfügung stehenden Gesamtfläche des Trägermaterials neben den Elektrodenkontaktflächen auch noch die Leiterbahnen, welche die Elektrodenkontaktflächen mit Elektrodenanschlussflächen verbinden, untergebracht werden müssen. Das bedeutet, dass für eine hoch aufgelöste Elektrodenstruktur nur ein kleiner Teil der Gesamtfläche des Trägermaterials für die Anordnung der Elektrodenkontaktflächen zur Verfügung steht. Hierbei gilt, dass bei gleichbleibender Gesamtfläche des Trägermaterials die für die Anordnung der Elektrodenkontaktflächen zur Verfügung stehende Fläche mit zunehmender Auflösung kleiner wird, weil mit zunehmender Auflösung auch die Anzahl der Leiterbahnen zunimmt.

Die Gesamtfläche des Trägermaterials kann allerdings nicht einfach vergrößert werden, weil bei einer Herstellung der Elektrodenkontaktflächen und Leiterbahnen mit einem Laser die im Bereich des Randes des Trägermaterials herzustellenden Elektrodenkontaktflächen und Leiterbahnen nicht mehr im Fokus des Lasers liegen. Die im Bereich des Randes des Trägermaterials herzustellenden Elektrodenkontaktflächen und Leiterbahnen können daher nicht mit derselben Genauigkeit hergestellt werden wie die im mittleren Bereich des Trägermaterials herzustellenden Elektrodenkontaktflächen und Leiterbahnen. Damit erhöht sich mit zunehmender Gesamtfläche des Trägermaterials auch die Wahrscheinlichkeit eines Fehlers während des Herstellungsprozesses, was einen größeren Ausschuss zur Folge hat.

Um das Problem der maximal möglichen Auflösung bei gleichbleibender Gesamtfläche des Trägermaterials zu umgehen, ist es bekannt, mehrschichtige Elektrodenstrukturen zu verwenden, wobei in einer oberen Schicht die Elektrodenkontaktflächen und Leiterbahnen so angeordnet werden, dass sich unterhalb dieser Elektrodenkontaktflächen und Leiterbahnen keine Elektrodenkontaktflächen der darunterliegenden Schichten befinden.

Um solche mehrschichtigen Elektrodenstrukturen herzustellen, ist es bekannt, diese schichtweise aufzubauen. Dabei wird zunächst auf einem ersten Trägermaterial eine Elektrodenstruktur mit Elektrodenkontaktflächen und Leiterbahnen erzeugt. Das Erzeugen der Elektrodenkontaktflächen und der Leiterbahnen kann beispielsweise mit Hilfe eines Lasers bewerkstelligt werden. In einem weiteren Schritt wird auf die so erzeugte Elektrodenstruktur ein weiteres Trägermaterial aufgetragen, auf welcher wiederum eine Elektrodenstruktur mit Elektrodenkontaktflächen und Leiterbahnen mit Hilfe eines Lasers erzeugt werden. Nach dem Erzeugen der zweiten Elektrodenstruktur wird eine weitere Trägerschicht aufgetragen. Die Schritte für das Erzeugen der zweiten Elektrodenstruktur und das Auftragen eines weiteren Trägermaterials können für weitere Elektrodenschichten wiederholt werden.

Dieses Verfahren weist allerdings den gravierenden Nachteil auf, dass bei der Herstellung einer oberen Elektrodenstruktur mittels eines Lasers darunterliegende Elektrodenstrukturen beschädigt werden können. Um dies zu vermeiden, können einerseits die Abstände der einzelnen Elektrodenstrukturen so gewählt werden, dass ein Beschädigen einer darunterliegenden Elektrodenstruktur mit dem Laser sicher verhindert wird. Andererseits kann die Anordnung der Elektrodenkontaktflächen und der Leiterbahnflächen einer oberen Elektrodenstruktur so gewählt werden, dass sich ein möglichst kleiner Anteil der darunterliegenden Elektrodenstruktur im Bearbeitungsbereich des Lasers zur Herstellung der oberen Elektrodenstruktur befindet.

Im ersteren Fall hat ein Vergrößern des Abstandes der einzelnen Elektrodenschichten allerdings den Nachteil, dass sich damit auch die Dicke der gesamten Elektrode vergrößert, was sich etwa bei der Implantation nachteilig auswirkt. Im letzteren Fall könnte nicht die gesamte Fläche zur Herstellung von Elektrodenkontaktflächen verwendet werden, so dass keine optimale Ausnutzung der Gesamtfläche des Trägermaterials erreicht wird. Zudem kann ein Beschädigen darunterliegender Elektrodenstrukturen damit nicht ausgeschlossen werden.

US 2010/0023102 A1 betrifft ein Verfahren zum Herstellen eines Neurostimulationsschaltkreises, bei welchem individuelle implantierbare Schichten durch einen Laser oder durch mechanische Mittel geschnitten werden und dann gestapelt werden, wodurch ein effizienteres Herstellungsverfahren für hochverdichtete implantierbare Elektrodenfelder und -kabel bereitgestellt werden sollen. Die einzelnen implantierbaren Schichten können verschmolzen und konglomeriert werden, um einen Neurostimulationsschaltkreis zu formen, bei welchem die Leiter und Anschlussstellen innerhalb eines kontinuierlichen Polymer-Isolierungsfilm eingekapselt sind.

### Aufgabe der vorliegenden Erfindung

Aufgabe der vorliegenden Erfindung ist es daher, ein Herstellungsverfahren für eine Elektrodenstruktur insbesondere für eine neuronale Schnittstelle anzugeben, welches die Herstellung besonders hoch auflösender Elektrodenstrukturen ermöglicht, ohne dabei die aus dem Stand der Technik bekannten Nachteile auf zuweisen. Des Weiteren ist es Aufgabe, eine derartige Elektrodenstruktur bereitzustellen.

### Erfindungsgemäße Lösung

Diese Aufgabe wird durch ein Verfahren zum Herstellen einer Elektrodenstruktur für neuronale Schnittstellen sowie durch eine Elektrodenstruktur für eine neuronale Schnittstelle nach den unabhängigen Ansprüchen gelöst. Vorteilhafte Ausgestaltungen und Weiterbildungen der Erfindung sind in den jeweiligen abhängigen Ansprüchen angegeben.

Erfindungsgemäß bereitgestellt wird also ein Verfahren zum Herstellen einer Elektrodenstruktur insbesondere für eine neuronale Schnittstelle, wobei das Verfahren das Herstellen eines ersten Elektrodenmoduls und wenigstens eines zweiten Elektrodenmoduls umfasst, wobei jeweils
- auf einem ersten Substrat eine Anzahl von Elektrodenkontaktflächen und eine Anzahl von Leiterbahnen erzeugt werden,
- auf dem ersten Substrat und den darauf erzeugten Elektrodenkontaktflächen und Leiterbahnen ein zweites Substrat aufgebracht wird, und
- in dem jeweiligen zweiten Substrat im Bereich der Elektrodenkontaktflächen Öffnungen hergestellt werden, und
wobei das Verfahren das Aufbringen des wenigstens einen zweiten Elektrodenmoduls auf das erste Elektrodenmodul umfasst, wobei das erste Substrat des zweiten Elektrodenmoduls auf dem zweiten Substrat des ersten Elektrodenmoduls angeordnet wird. Hierbei werden in dem wenigstens einem zweiten Elektrodenmodul Löcher erzeugt, welche jeweils das erste Substrat und das zweite Substrat des zweiten Elektrodenmoduls durchdringen, wobei die Löcher so angeordnet werden, dass sie beim Aufbringen des zweiten Elektrodenmoduls auf das erste Elektrodenmodul jeweils mit wenigstens einer Elektrodenkontaktfläche des ersten Elektrodenmoduls korrespondieren.

Die einzelnen Elektrodenmodule, die jeweils ein erstes Substrat, Elektrodenkontaktflächen und Leiterbahnen sowie darüber ein zweites Substrat umfassen, werden also getrennt voneinander hergestellt und erst danach zu der Elektrodenstruktur zusammengefügt. Dieses Verfahren hat zunächst den Vorteil, dass z.B. bei einem Produktionsfehler bei der Herstellung eines der Elektrodenmodule nur dieses eine Elektrodenmodul unbrauchbar wird, nicht aber die übrigen. Die einzelnen Elektrodenmodule können also unmittelbar nach ihrer Herstellung auf Fehler überprüft werden und dann aussortiert werden, bevor sie zur Elektrodenstruktur zusammengefügt werden. Die Modularisierung der Herstellung bringt weitere Vorteile mit sich. So wird die Herstellung der Elektrodenstruktur vereinfacht. Zudem können hoch auflösende Elektrodenmodule hergestellt werden, welche zusammen eine besonders hoch auflösende Elektrodenstruktur bilden. Aufgrund der Modularisierung könnten häufiger benötigte Teile könnten vorentworfen und gefertigt werden und so in einem Baukastensystem zu Bildung von komplexere Elektrodenstrukturen bereitgehalten werden.

Es entsteht eine sehr flexible Elektrodenstruktur, die sich hervorragend zur Bildung von flächenförmigen Sensoren eignet, die z.B. in ein Gehirn implantierbar sind.

Die Elektrodenkontaktflächen und die Leiterbahnen des wenigstens einen zweiten Elektrodenmoduls werden bevorzugt so erzeugt, dass sie beim Aufbringen des zweiten Elektrodenmoduls auf das erste Elektrodenmodul die Elektrodenkontaktflächen des ersten Elektrodenmoduls nicht abdecken.

Die Elektrodenkontaktflächen des ersten Elektrodenmoduls und des wenigstens einen zweiten Elektrodenmoduls bilden zusammen die Elektrodenkontaktflächen der Elektrodenstruktur. Dadurch kann in vorteilhafter Weise die Gesamtfläche der Elektrodenstruktur für die Anordnung von Elektrodenkontaktflächen ausgenutzt werden.

Die Elektrodenauflösung des ersten Elektrodenmoduls kann verschieden von der Elektrodenauflösung des wenigstens einen zweiten Elektrodenmoduls sein, wobei die Elektrodenauflösung als die Anzahl der Elektrodenkontaktflächen pro Flächeneinheit definiert ist.

Dadurch wird es möglich, eine Elektrodenstruktur mit lokal unterschiedlichen Auflösungen bereitzustellen. So muss, wenn z.B. auf einem Teil der Oberfläche der Elektrodenstruktur eine sehr viel höhere Auflösung (oder Anzahl von Elektrodenkontaktflächen) gefordert wird als auf einem anderen, nur dieser Teil durch ein Elektrodenmodul gebildet werden, das mit einer höheren Präzision gefertigt wird als der andere Teil. Diese Aufteilung bringt erhebliche Vorteile in den Produktionskosten mit sich, denn mit der geforderten Präzision (oder Auflösung) steigen der Herstellungsaufwand und die Ausschussrate.

Es hat sich als vorteilhaft herausgestellt, vor dem Aufbringen des wenigstens einen zweiten Elektrodenmoduls auf das erste Elektrodenmodul auf dem zweiten Substrat des ersten Elektrodenmoduls ein Lösungsmittel aufzubringen.

Dadurch kann das wenigstens eine zweite Elektrodenmodul exakt auf dem ersten Elektrodenmodul ausgerichtet werden.

In wenigstens einem der Elektrodenmodule können Kanäle erzeugt werden, welche jeweils eine Eintrittsöffnung und wenigstens eine Austrittsöffnung aufweisen, zum Transport eines Fluid. Derartige mikrofluidische Kanäle können zur gezielten Medikamentenabgabe vorgesehen sein.

Das erste Substrat und/oder das zweite Substrat werden vorzugsweise aus der Gruppe der synthetischen Polymere, besonders bevorzugt aus der Gruppe der Poly(organo)siloxane ausgewählt.

Das erste Elektrodenmodul und das wenigstens eine zweite Elektrodenmodul können rechnergestützt entworfen werden.

Die auf dem ersten Substrat zu erzeugenden Elektrodenkontaktflächen und Leiterbahnen können im Laserschneidverfahren auf dem ersten Substrat erzeugt werden. Die mikrofluidischen Kanäle können ebenfalls im Laserschneidverfahren hergestellt werden.

In wenigstens einem der Elektrodenmodule kann wenigstens ein Lichtwellenleiter integriert werden, welcher an dem zweiten Substrat nach außen geführt wird.

Bereitgestellt wird auch eine Elektrodenstruktur insbesondere für eine neuronale Schnittstelle, umfassend ein erstes Elektrodenmodul und wenigstens ein zweites Elektrodenmodul, welche jeweils ein erstes Substrat, auf welchem eine Anzahl von Elektrodenkontaktflächen und eine Anzahl von Leiterbahnen angeordnet sind, und ein zweites Substrat, welches auf dem ersten Substrat und den darauf angeordneten Elektrodenkontaktflächen und Leiterbahnen angeordnet ist, aufweisen, wobei das wenigstens eine zweite Elektrodenmodul auf dem ersten Elektrodenmodul angeordnet ist, wobei in dem wenigstens einen zweiten Elektrodenmodul Löcher angeordnet sind, welche jeweils das erste Substrat und das zweite Substrat des zweiten Elektrodenmoduls durchdringen, wobei die Löcher so angeordnet sind, dass sie jeweils mit wenigstens einer Elektrodenkontaktfläche des ersten Elektrodenmoduls korrespondieren.

Die Elektrodenkontaktflächen und die Leiterbahnen des wenigstens einen zweiten Elektrodenmoduls können so angeordnet sein, dass sie die Elektrodenkontaktflächen des ersten Elektrodenmoduls nicht abdecken.

Die Elektrodenauflösung des ersten Elektrodenmoduls kann verschieden von der Elektrodenauflösung des wenigstens einen zweiten Elektrodenmoduls sein, wobei die Elektrodenauflösung als die Anzahl der Elektrodenkontaktflächen pro Flächeneinheit definiert ist.

Es sei darauf hingewiesen, dass die einzelnen Elektrodenmodule in ihrer Größe, d.h. Oberfläche, nicht deckungsgleich sein müssen. Elektrodenmodule können auch nebeneinander auf ein darunterliegendes Elektrodenmodul gesetzt werden.

### Kurzbeschreibung der Figuren

Weitere Einzelheiten und Merkmale der Erfindung sowie konkrete Ausführungbeispiele der Erfindung ergeben sich aus der nachfolgenden Beschreibung in Verbindung mit der Zeichnung. Es zeigt:
- Fig. 1: einen Ausschnitt eines ersten Elektrodenmoduls und eines zweiten Elektrodenmoduls einer erfindungsgemäßen Elektrodenstruktur im Querschnitt;
- Fig. 2: einen Ausschnitt von zwei Elektrodenmodulen einer erfindungsgemäßen Elektrodenstruktur im zusammengesetzten Zustand im Querschnitt;
- Fig. 3: ein konkretes Beispiel von zwei Elektrodenmodulen, welche zu einer Elektrodenstruktur zusammengesetzt werden, wobei die beiden Elektrodenmodule jeweils die gleiche Auflösung aufweisen;
- Fig. 4: zwei Elektrodenmodule, welche zu einer erfindungsgemäßen Elektrodenstruktur zusammengesetzt werden, wobei die beiden Elektrodenmodule jeweils eine unterschiedliche Auflösung aufweisen; und
- Fig. 5: ein weiteres Ausführungsbeispiel der vorliegenden Erfindung.

### Detaillierte Beschreibung der Erfindung

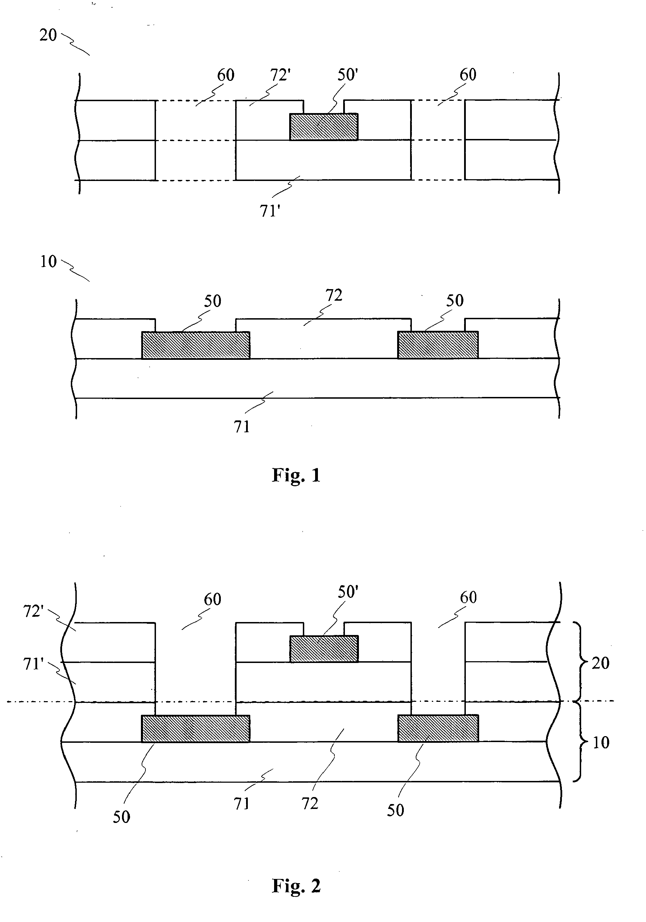

**Fig. 1** zeigt ein erstes Elektrodenmodul 10 und ein zweites Elektrodenmodul 20, welche jeweils getrennt voneinander hergestellt werden und nach der Herstellung erfindungsgemäß zusammengesetzt werden, um dann die endgültige Elektrodenstruktur zu bilden.

Es wird zunächst angegeben, wie die einzelnen Elektrodenmodule 10, 20 hergestellt werden, bevor mit Bezug auf Fig. 2 angegeben wird, wie aus den einzelnen Elektrodenmodulen eine endgültige Elektrodenstruktur gemäß der Erfindung hergestellt wird.

Zur Herstellung des ersten Elektrodenmoduls wird zunächst auf einem Objektträger, etwa eine Glasscheibe, ein Klebeband aufgewalzt. Das Klebeband ist dazu vorgesehen, damit das erste Elektrodenmodul 10 am Ende des Herstellungsprozesses leicht von diesem abgelöst werden kann. Anstelle eines Klebebandes kann auch eine Folie aus ausgehärtetem Silikon verwendet werden.

Auf das Klebeband wird ein erstes Substrat 71, welches hier mit Heptan, vorzugsweise mit n-Heptan verdünntes Silikon ist, aufgeschleudert. Diese Silikonschicht bildet die untere Silikonschicht 71, auf welcher die Elektrodenkontaktflächen und die Leiterbahnen der Elektrodenstruktur des ersten Elektrodenmoduls 10 erzeugt werden. Die untere Silikonschicht 71 des ersten Elektrodenmoduls 10 wird an der Anschlussseite mit einem Laser strukturiert, um im weiteren Herstellungsverfahren Metallstrukturen zu erhalten, an welche beispielsweise Verbindungskabel angebracht werden können.

Nachdem die aufgeschleuderte untere Silikonschicht 71 hinreichend weit ausgehärtet ist, wird auf die untere Silikonschicht 71 eine Metallfolie aufgewalzt. Die Metallfolie kann etwa Platin, Edelstahl oder eine Legierung auf Nickel-Kobalt-Basis (z.B. MP35N-Legierung) aufweisen. Die Dicke der Metallfolie kann zwischen etwa 5 ₁₁m und 50 µm betragen.

In einem weiteren Schritt wird die aufgewalzte Metallfolie mit einem Laser strukturiert, um Elektrodenkontaktflächen 50 und Leiterbahnen 30 zu bilden. Herausgetrennte Metallreste werden von der unteren Silikonschicht 71 entfernt. Dies kann manuell mit Hilfe einer Pinzette erfolgen. Das Entfernen der Metallreste kann aber auch maschinell erfolgen.

Sobald die erforderliche Elektrodenstruktur, d.h. die Elektrodenkontaktflächen 50 und die Leiterbahnen 30, auf der unteren Silikonschicht 71 fertig hergestellt sind, wird auf die untere Silikonschicht 71 und auf die auf der unteren Silikonschicht 71 erzeugte Elektrodenstruktur ein zweites Substrat, vorzugsweise eine zweite Silikonschicht 72, aufgeschleudert. Die zweite Silikonschicht 72 wird vorzugsweise ebenfalls mit n-Heptan verdünnt.

Nach dem Aushärten der zweiten Silikonschicht 72 werden Öffnungen erzeugt, um die Elektrodenkontaktflächen freizulegen, so dass bei einem späteren Gebrauch der erfindungsgemäßen Elektrodenstruktur ein Kontakt zwischen einem Gewebe und der Elektrodenkontaktfläche hergestellt werden kann. Das Erzeugen dieser Öffnungen wird hier ebenfalls mit Hilfe des Lasers bewerkstelligt. Die Oberseite der an der Anschlussseite verlaufenden Metallstrukturen, an welchen etwa ein Kabel angeschlossen werden kann, kann ebenfalls mit Hilfe des Lasers freigelegt werden.

Alternativ können die Metallstrukturen an der Anschlussseite auch mit Hilfe einer Polyamidfolie (z.B. Kaptonfolie) freigelegt werden, indem vor dem Auf bringen der oberen Silikonschicht 72 eine strukturierte Maske aus Polyamid an der Anschlussseite auf die Metallstrukturen aufgebracht wird. Die Polyamidfolie kann ebenfalls mit Hilfe eines Lasers strukturiert werden. Nach dem Aufbringen der oberen Silikonschicht 72 kann die strukturierte Polyamidfolie zusammen mit dem auf ihr aufgebrachten Silikon entfernt werden.

In einem abschließenden Schritt kann mit dem Laser noch eine Umrandung erzeugt werden, indem mit dem Laser ein umlaufender Schnitt erzeugt wird, welcher sowohl die obere Silikonschicht 72 als auch die untere Silikonschicht 71 durchschneidet.

Das so erzeugte erste Elektrodenmodul 10 kann nun von dem Klebeband abgelöst werden. Das erste Elektrodenmodul 10 weist nun eine Anzahl von Elektrodenkontaktflächen 50 und eine Anzahl von Leiterbahnen 30 auf, wobei die obere Silikonschicht 72 im Bereich der Elektrodenkontaktflächen 50 Öffnungen auf weist, so dass die Elektrodenkontaktflächen 50 freigelegt sind. Die Dicke des ersten Elektrodenmoduls 10 beträgt zwischen 50 ₁₁m und 150 ₁₁m.

Das zweite Elektrodenmodul 20 wird im Wesentlichen mit demselben Verfahren hergestellt wie das erste Elektrodenmodul 10. Die Elektrodenkontaktflächen 50' und die Leiterbahnen 30' des zweiten Elektrodenmoduls 20 werden so angeordnet, dass sie die Elektrodenkontaktflächen 50 des ersten Elektrodenmoduls 10 nicht abdecken, wenn das zweite Elektrodenmodul 20 auf dem ersten Elektrodenmodul 10 aufgebracht wird, wie mit Bezug auf Fig. 2 näher beschrieben wird.

Die Elektrodenkontaktflächen 50' bzw. die Leiterbahnen 30' des zweiten Elektrodenmoduls 20 können aber so angeordnet werden, dass sie nach dem Aufbringen des zweiten Elektrodenmoduls 20 auf das erste Elektrodenmodul 10 die Leiterbahnen 30 des ersten Elektrodenmoduls 10 teilweise oder vollständig abdecken. Dadurch wird in vorteilhafter Weise erreicht, dass im Gegensatz zu einschichtigen Elektrodenstrukturen nahezu die gesamte Fläche des Elektrodenträgers zur Anordnung von Elektrodenkontaktflächen 50, 50' zur Verfügung steht, weil auf dem zweiten Elektrodenmodul 20 die Elektrodenkontaktflächen 50' auch dort angeordnet werden können, wo bei dem ersten Elektrodenmodul die Leiterbahnen 30 verlaufen.

Zusätzlich werden in dem zweiten Elektrodenmodul 20 Löcher 60 erzeugt, welche das zweite Elektrodenmodul 20 vollständig durchdringen. Die Löcher 60 werden so an dem zweiten Elektrodenmodul 20 angeordnet, dass sie nach dem Aufbringen des zweiten Elektrodenmoduls 20 auf das erste Elektrodenmodul 10 mit den Elektrodenkontaktflächen 50 des ersten Elektrodenmoduls 10 korrespondieren. Damit wird sichergestellt, dass nach dem Aufbringen des zweiten Elektrodenmoduls 20 auf das erste Elektrodenmodul 10 die Elektrodenkontaktflächen 50 des ersten Elektrodenmoduls 10 weiterhin frei liegen. Die Dicke des zweiten Elektrodenmoduls 20 beträgt zwischen 50 µm und 150 ₁₁m.

Die Löcher 60 können mit Hilfe des Lasers erzeugt werden. Alternativ können die Löcher 60 auch in einem Stanzverfahren hergestellt werden.

Das getrennte Herstellen der beiden Elektrodenmodule 10, 20 hat den wesentlichen Vorteil, dass beide Elektrodenmodule im Wesentlichen mit dem gleichen Herstellungsverfahren hergestellt werden können, ohne dass dabei darunterliegende Elektrodenstrukturen, etwa des ersten Elektrodenmoduls 10, beschädigt werden.

Die Anordnung der Elektrodenkontaktflächen 50, 50', der Leiterbahnen 30, 30' sowie der Elektrodenanschlussflächen 40, 40' der beiden Elektrodenmodule 10, 20 kann etwa mit Hilfe einer CAD-Software geplant und getestet werden. Durch die Verwendung eines Lasers zum Erzeugen der Elektrodenstrukturen auf dem ersten und dem zweiten Elektrodenmodul können zum Einen Objektgrößen von bis herunter zu 50 µm erzeugt werden, und zum Anderen können die Elektrodenkontaktflächen bzw. die Leiterbahnen so exakt erzeugt werden, dass eine Anordnung des zweiten Elektrodenmoduls 20 auf das erste Elektrodenmodul 10 wie geplant erfolgen kann.

**Fig. 2** zeigt eine erfindungsgemäße Elektrodenstruktur mit einem ersten Elektrodenmodul 10 und einem zweiten Elektrodenmodul 20, welche miteinander verbunden sind. Die beiden Elektrodenmodule 10, 20 werden wie mit Bezug auf Fig. 1 beschrieben hergestellt. Vor dem Zusammenfügen der beiden Elektrodenmodule 10, 20 bzw. vor dem Anordnen des zweiten Elektrodenmoduls 20 auf dem ersten Elektrodenmodul 10 wird an der oberen Silikonschicht 72 des ersten Elektrodenmoduls 10 ein Lösungsmittel aufgebracht, welches eine genaue Justierung des zweiten Elektrodenmoduls 20 auf dem ersten Elektrodenmodul 10 ermöglicht. Als Lösungsmittel kann etwa Methanol verwendet werden. Methanol als Lösungsmittel hat den Vorteil, dass die obere Silikonschicht 72 des ersten Elektrodenmoduls 10 und die untere Silikonschicht 71' des zweiten Elektrodenmoduls 20 nur sehr gering aufquellen.

Nach dem Aufbringen des zweiten Elektrodenmoduls 20 auf das erste Elektrodenmodul 10 werden die Elektrodenmodule 10, 20 unlösbar miteinander verbunden. Das kann beispielsweise im Plasmabond-Verfahren erfolgen.

Dabei wird die Unterseite des zweiten Elektrodenmoduls 20 im Sauerstoffplasma aktiviert, wobei die Unterseite reaktiv wird. Das zweite Elektrodenmodul 20 wird dann auf das mit dem Lösungsmittel versehene erste Elektrodenmodul 10 aufgebracht. Das Lösungsmittel erlaubt ein genaues Ausrichten des zweiten Elektrodenmoduls 20 auf dem ersten Elektrodenmodul 10. Das Lösungsmittel verhindert zudem, dass sich zwischen den Elektrodenmodulen Luftblasen bilden. Sobald das Lösungsmittel verdampft ist, sind die Elektrodenmodule 10, 20 unlösbar miteinander verbunden. Das Verdampfen des Lösungsmittels benötigt etwa 10 Minuten. Während dieser Zeit kann das zweite Elektrodenmodul 20 auf dem ersten Elektrodenmodul 10 justiert werden.

Das unlösbare Verbinden der beiden Elektrodenmodule 10, 20 kann auch mit anderen geeigneten Verfahren bzw. Mitteln durchgeführt werden.

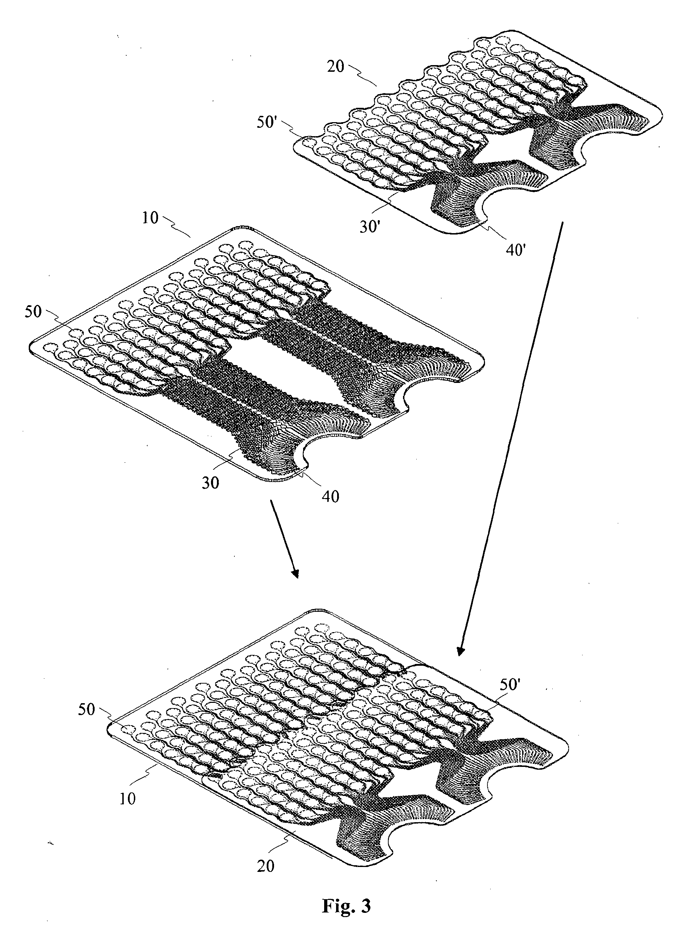

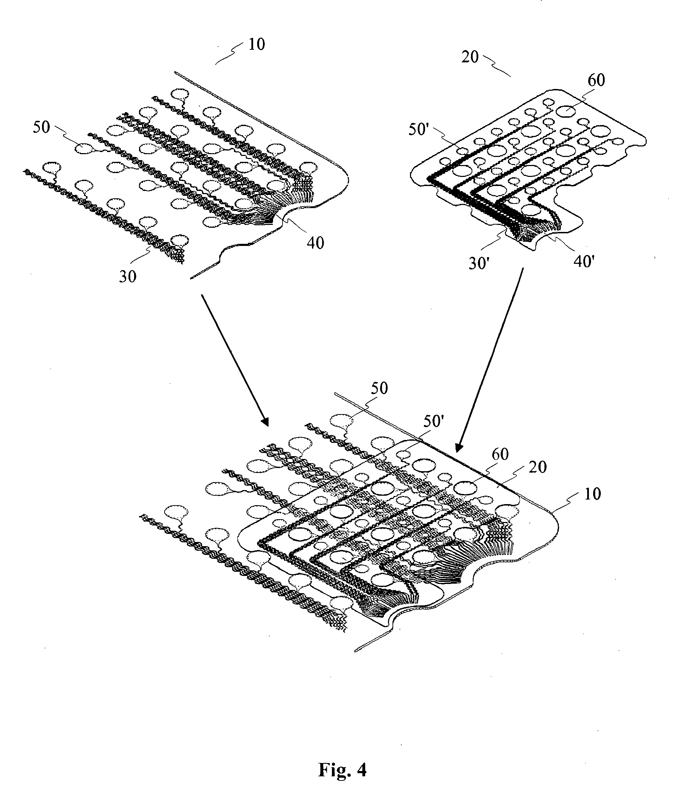

Wie aus Fig. 2 ersichtlich, können die Elektrodenkontaktflächen 50 und 50' des ersten Elektrodenmoduls 10 bzw. des zweiten Elektrodenmoduls 20 eine unterschiedliche Fläche aufweisen, d.h. sie können unterschiedlich groß sein. Auch können die einzelnen Elektrodenkontaktflächen eines Elektrodenmoduls eine unterschiedliche Fläche aufweisen. Damit kann eine Elektrodenstruktur mit unterschiedlichen Auflösungen hergestellt werden, wie mit Bezug auf die Fig. 3 und 4 näher beschrieben wird.

Auf dem ersten Elektrodenmodul 10 können auch mehrere zweite Elektrodenmodule 20 nebeneinander angeordnet werden. Die nebeneinander angeordneten Elektrodenmodule 20 können dabei jeweils eine verschiedene Auflösung aufweisen. Dies hat den Vorteil, dass die zweiten Elektrodenmodule 20 jeweils mit einer Auflösung hergestellt werden können und anschließend zu einer zweiten Elektrodenschicht mit unterschiedlichen Auflösungen zusammengesetzt werden können.

Fig. 3 zeigt eine Elektrodenstruktur, welche durch Zusammensetzen eines ersten Elektrodenmoduls 10 und eines zweiten Elektrodenmoduls 20 hergestellt wird. Die Elektrodenmodule 10, 20 weisen hierbei jeweils die gleiche Auflösung auf.

Das erste Elektrodenmodul 10 weist eine Anzahl von Elektrodenkontaktflächen 50 auf, welche jeweils über eine Leiterbahn 30 mit Elektrodenanschlussflächen 40 verbunden sind.

Das zweite Elektrodenmodul 20 weist ebenfalls eine Anzahl von Elektrodenkontaktflächen 50' auf, welche über Leiterbahnen 30' mit einer Anzahl von Elektrodenanschlussflächen 40' verbunden sind.

Die Anordnung der Elektrodenkontaktflächen 50' auf dem zweiten Elektrodenmodul 20 ist hier so gewählt, dass diese nach dem Zusammenfügen bzw. nach dem Anordnen des zweiten Elektrodenmoduls 20 auf dem ersten Elektrodenmodul 10 sich im Wesentlichen im Bereich der Leiterbahnen 30 des ersten Elektrodenmoduls 10 befinden. Wie aus Fig. 3 besonders gut erkennbar ist, kann die Auflösung der Elektrodenstruktur durch Aufbringen des zweiten Elektrodenmoduls 20 auf das erste Elektrodenmodul 10 verdoppelt werden.

**Fig. 4** zeigt ein weiteres konkretes Beispiel einer erfindungsgemäßen Elektrodenstruktur, bei welchem ein erstes Elektrodenmodul 10 und ein zweites Elektrodenmodul 20 zusammengefügt werden. In dem in Fig. 4 gezeigtem Beispiel ist die Auflösung des ersten Elektrodenmoduls 10 verschieden von der Auflösung des zweiten Elektrodenmoduls 10. An dem ersten Elektrodenmodul 10 sind eine Anzahl von Elektrodenkontaktflächen 50 angeordnet, welche jeweils über eine Leiterbahn 30 mit Elektrodenanschlussflächen 40 verbunden sind. An dem zweiten Elektrodenmodul 20 sind eine Anzahl von Elektrodenkontaktflächen 50' angeordnet, welche ebenfalls über Leiterbahnen 30' mit Elektrodenanschlussflächen 40' verbunden sind. Die Elektrodenkontaktflächen 50' des zweiten Elektrodenmoduls 20 sind hier kleiner gewählt als die Elektrodenkontaktflächen 50 des ersten Elektrodenmoduls 10.

Zusätzlich sind in dem zweiten Elektrodenmodul 20 Löcher 60 vorgesehen, welche so angeordnet sind, dass sie im Wesentlichen mit den Elektrodenkontaktflächen 50 des ersten Elektrodenmoduls korrespondieren, so dass eine Kontaktierung der Elektrodenkontaktflächen 50 des ersten Elektrodenmoduls mit einem Gewebe auch nach dem Aufbringen des zweiten Elektrodenmoduls 20 auf das erste Elektrodenmodul 10 gewährleistet ist.

Zusätzlich oder alternativ zu den Elektrodenkontaktflächen 50, 50' bzw. Leiterbahnen 30, 30' können in einem der Elektrodenmodule 10, 20 auch Kanäle vorgesehen sein, welche beispielsweise im Anschlussbereich eine Eintrittsöffnung und an der oberen Silikonschicht 72 bzw. 72' eine Austrittsöffnung aufweisen. Derartige mikrofluidische Kanäle können etwa dazu vorgesehen sein, um eine zielgerichtete Medikamentenabgabe durchzuführen. So kann beispielsweise ein Elektroden-Implantat mit zwei Modulen hergestellt werden, wobei ein erstes Modul als Elektrodenmodul ausgestaltet ist und ein zweites Modul zusätzlich oder alternativ zu den Elektrodenkontaktflächen eine Anzahl von mikrofluidischen Kanälen, etwa zur Medikamentenabgabe, aufweist.

Die für die Erzeugung derartiger mikrofluidischer Kanäle erforderlichen Verfahrensschritte können in das mit Bezug auf Fig. 1 erläuterte Verfahren integriert werden.

Alternativ können die mikrofluidischen Kanäle auch zwischen zwei Elektrodenmodulen erzeugt werden. So können beispielsweise nach dem Aufbringen der oberen Silikonschicht 72 des ersten Elektrodenmoduls 10 mit Hilfe des Lasers in der oberen Silikonschicht 72 die entsprechenden Kanäle erzeugt werden. In dem zweiten Elektrodenmodul 20 können ebenfalls mit Hilfe des Lasers entsprechende Austrittsöffnungen hergestellt werden, welche mit den Kanälen auf dem ersten Elektrodenmodul korrespondieren. Nach dem Aufbringen des zweiten Elektrodenmoduls 20 auf das erste Elektrodenmodul 10 werden dann im Wesentlichen geschlossene mikrofluidische Kanäle mit Eintritts- und Austrittsöff nungen gebildet.

Zusätzlich oder alternativ zu den mikrofluidischen Kanälen können auch optische Module in die Elektrodenmodule 10 bzw. 20 integriert werden. Beispielsweise können in das Elektrodenmodul 20 Lichtwellenleiter integriert werden, welche an der oberen Silikonschicht 72' sowie an der Anschlussseite des Elektrodenmoduls 20 nach außen geführt werden. Mit Hilfe der integrierten Lichtwellenleiter können bestimmte Gehirnregionen zusätzlich stimuliert werden.

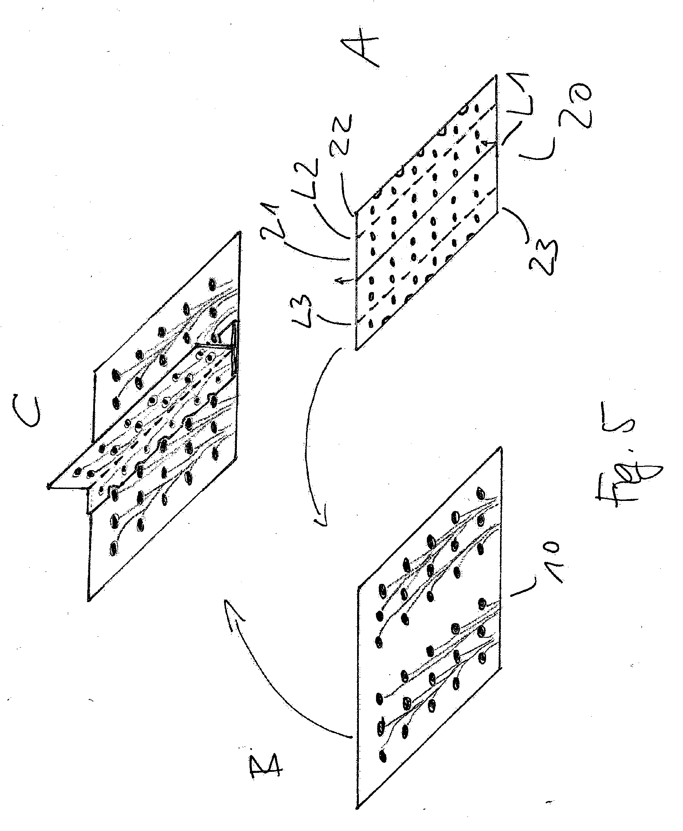

Die Erfindung lässt sich zur Bildung einer dreidimensionalen Elektrodenstruktur erweitern. Fig. 5 illustriert, wie eine derartige dreidimensionale Elektronenstruktur hergestellt werden kann. Vor dem Aufbringen eines zweiten Elektrodenmoduls 20 auf das erste Elektrodenmodul 10 wird das zweite Elektrodenmodul 20 zunächst entlang der Linie L1 umgefaltet, und zwar so, dass die obere Silikonschicht (zweites Substrat) 72' nach außen zeigt und die untere Silikonschicht (erstes Substrat) 71' nach innen orientiert ist, vgl. Fig. 5A. Wenn die Linie All in der Mitte des Elektrodenmoduls 20 verläuft, entsteht ein gefaltetes Elektrodenmodul 20 halber Größe. Sodann werden die so entstehenden Seiten des Elektrodenmoduls 20 entlang der Linien L2 und L3 aufgeknickt, derart, dass die resultierende Form im Profil ein umgekehrtes T mit zwei Halbschenkeln 22 und 23 und einer Senkrechten 21 darstellt. Schließlich wird das gefaltete und geknickte zweite Elektrodenmodul 20 (als umgekehrtes T, also "kopfüber") auf das erste Elektrodenmodul 10 (Fig. 5B) aufgebracht, wie zuvor beschrieben. Es entsteht eine dreidimensionale Trägerstruktur, wie sie in Fig. 5C dargestellt ist.

Selbstverständlich muss das T-förmige Profil nicht symmetrisch sein, Faltungen und Knickungen entlang anderer Linien sind denkbar, die Proportionen der Flächen können anders als in Fig. 5 illustriert sein.

Die dreidimensionale Struktur eignet sich in besonderer Weise dann, wenn Signale einander gegenüber liegender Hirnareale erfasst werden sollen und z.B. der Raum für zwei separate Strukturen nicht ausreicht.

Die Erfindung umfasst auch den rechnergestützten Entwurf einer Elektrodenstruktur insbesondere für eine neuronale Schnittstelle, wobei das Verfahren das Entwerfen eines ersten Elektrodenmoduls und wenigstens eines zweiten Elektrodenmoduls umfasst, wobei jeweils
- auf einem ersten Substrat eine Anzahl von Elektrodenkontaktflächen und eine Anzahl von Leiterbahnen erzeugt werden,
- auf dem ersten Substrat und den darauf erzeugten Elektrodenkontaktflächen und Leiterbahnen ein zweites Substrat aufgebracht wird, und
- in dem jeweiligen zweiten Substrat im Bereich der Elektrodenkontaktflächen Öffnungen hergestellt werden, und
wobei das Verfahren das Entwerfen des Aufbringens des wenigstens einen zweiten Elektrodenmoduls auf das erste Elektrodenmodul umfasst, wobei das erste Substrat des zweiten Elektrodenmoduls auf dem zweiten Substrat des ersten Elektrodenmoduls angeordnet wird.

Die Elektrodenstruktur gemäß dem Entwurf kann dann mittels des oben beschriebenen Verfahrens hergestellt werden. Zuvor kann der Entwurf rechnergestützt getestet werden, z.B. können Simulationen durchgeführt werden.

Alle vorteilhaften Weiterbildungen beziehen sich auch auf den rechnergestützten Entwurf.

### Bezugszeichenliste

- 10: erstes Elektrodenmodul (erste Elektrodenschicht)
- 20: zweites Elektrodenmodul (zweite Elektrodenschicht)
- 21: Senkrechte
- 22: Halbschenkel(-fläche)
- 23: Halbschenkel(-fläche)
- 30, 30': Leiterbahnen
- 40, 40': Elektrodenanschlussflächen
- 50, 50': (geöffnete) Elektrodenkontaktflächen
- 60: Löcher
- 71, 71': untere Silikonschicht (erstes Substrat)
- 72, 72': obere Silikonschicht (zweites Substrat)
- L1: Faltungslinie
- L2: Knicklinie
- L3: Knicklinie

## Patentansprüche

1. Verfahren zum Herstellen einer Elektrodenstruktur für eine neuronale Schnittstelle, wobei das Verfahren umfasst:
- Herstellen eines ersten Elektrodenmoduls (10) und wenigstens eines zweiten Elektrodenmoduls (20), wobei für das erste Elektrodenmoduls (10) und für wenigstens ein zweites Elektrodenmoduls (20) jeweils
- auf einem ersten Substrat (71, 71') eine Anzahl von Elektrodenkontaktflächen (50, 50') und eine Anzahl von Leiterbahnen (30, 30') erzeugt werden,
- auf dem ersten Substrat (71, 71') und den darauf erzeugten Elektrodenkontaktflächen (50, 50') und Leiterbahnen (30, 30') ein zweites Substrat (72, 72') aufgebracht wird, und
- in dem jeweiligen zweiten Substrat (72, 72') im Bereich der Elektrodenkontaktflächen (50, 50') Öffnungen hergestellt werden, und
- Aufbringen des wenigstens einen zweiten Elektrodenmoduls (20) auf das erste Elektrodenmodul (10), wobei das erste Substrat (71') des wenigstens einen zweiten Elektrodenmoduls (20) auf dem zweiten Substrat (72) des ersten Elektrodenmoduls (10) angeordnet wird,
**dadurch gekennzeichnet, dass** in dem wenigstens einen zweiten Elektrodenmodul (20) Löcher (60) erzeugt werden, welche jeweils das erste Substrat (71') und das zweite Substrat (72') des zweiten Elektrodenmoduls (20) durchdringen, wobei die Löcher (60) so angeordnet werden, dass sie beim Aufbringen des zweiten Elektrodenmoduls (20) auf das erste Elektrodenmodul (10) jeweils mit wenigstens einer Elektrodenkontaktfläche (50) des ersten Elektrodenmoduls (10) korrespondieren.

2. Verfahren nach Anspruch 1, wobei die Elektrodenkontaktflächen (50') und die Leiterbahnen (30') des wenigstens einen zweiten Elektrodenmoduls (20) so erzeugt werden, dass sie beim Aufbringen des zweiten Elektrodenmoduls (20) auf das erste Elektrodenmodul (10) die Elektrodenkontaktflächen (50) des ersten Elektrodenmoduls (10) nicht abdecken.

3. Verfahren nach einem der vorhergehenden Ansprüche, wobei die Elektrodenauflösung des ersten Elektrodenmoduls (10) verschieden von der Elektrodenauflösung wenigstens eines zweiten Elektrodenmoduls (20) gewählt wird, wobei die Elektrodenauflösung als die Anzahl der Elektrodenkontaktflächen pro Flächeneinheit definiert ist.

4. Verfahren nach einem der vorhergehenden Ansprüche, wobei vor dem Aufbringen des wenigstens einen zweiten Elektrodenmoduls (20) auf das erste Elektrodenmodul (10) auf dem zweiten Substrat (72) des ersten Elektrodenmoduls (10) ein Lösungsmittel aufgebracht wird.

5. Verfahren nach einem der vorhergehenden Ansprüche, wobei in wenigstens einem der Elektrodenmodule (10, 20), Kanäle erzeugt werden, welche jeweils eine Eintrittsöffnung und wenigstens eine Austrittsöffnung aufweisen, zum Transport eines Fluid.

6. Verfahren nach einem der vorhergehenden Ansprüche, wobei das erste Substrat (71, 71') und/oder das zweite Substrat (72, 72') aus der Gruppe der synthetischen Polymere, vorzugsweise aus der Gruppe der Poly(organo)siloxane ausgewählt wird.

7. Verfahren nach einem der vorhergehenden Ansprüche, wobei das erste Elektrodenmodul (10) und/oder das wenigstens eine zweite Elektrodenmodul (20) rechnergestützt entworfen werden.

8. Verfahren nach einem der vorhergehenden Ansprüche, wobei die auf dem ersten Substrat (71, 71') zu erzeugenden Elektrodenkontaktflächen (50, 50') und Leiterbahnen (30, 30') im Laserschneidverfahren auf dem ersten Substrat (71, 71') erzeugt werden.

9. Verfahren nach einem der vorhergehenden Ansprüche, wobei in wenigstens einem der Elektrodenmodule (10, 20) wenigstens ein Lichtwellenleiter integriert wird, welcher an dem zweiten Substrat (72, 72') nach außen geführt wird.

10. Verfahren nach einem der vorhergehenden Ansprüche, wobei vor dem Aufbringen des wenigstens einen zweiten Elektrodenmoduls (20) auf das erste Elektrodenmodul (10) wenigstens ein zweites Elektrodenmodul (20) wenigstens einmal entlang einer Linie (L1) gefaltet wird.

11. Verfahren nach Anspruch 10, wobei vor dem Aufbringen des wenigstens einen zweiten Elektrodenmoduls (20) auf das erste Elektrodenmodul (10) wenigstens ein zweites Elektrodenmodul (20) einmal entlang einer ersten Linie (L1) gefaltet wird, derart, dass das zweite Substrat (72') nach außen zeigt, und das wenigstens eine gefaltete zweite Elektrodenmodul (20) entlang einer zweiten Linie (L2) und entlang einer dritten Linie (L3) geknickt wird, derart, dass das resultierende wenigstens eine zweite Elektrodenmodul (20) im Profil ein umgekehrtes T-Profil darstellt.

12. Elektrodenstruktur insbesondere für eine neuronale Schnittstelle, umfassend ein erstes Elektrodenmodul (10) und wenigstens ein zweites getrennt von dem ersten Elektrodenmodul hergestelltes Elektrodenmodul (20), welche jeweils ein erstes Substrat (71, 71'), auf welchem eine Anzahl von Elektrodenkontaktflächen (50, 50') und eine Anzahl von Leiterbahnen (30, 30') angeordnet sind, und ein zweites Substrat (72, 72'), welches auf dem ersten Substrat (71, 71') und den darauf angeordneten Elektrodenkontaktflächen (50, 50') und Leiterbahnen (30, 30') angeordnet ist, aufweisen, wobei das wenigstens eine zweite Elektrodenmodul (20) auf dem ersten Elektrodenmodul (10) angeordnet ist,
**dadurch gekennzeichnet, dass** in dem wenigstens einen zweiten Elektrodenmodul (20) Löcher (60) angeordnet sind, welche jeweils das erste Substrat (71') und das zweite Substrat (72') des zweiten Elektrodenmoduls (20) durchdringen, wobei die Löcher (60) so angeordnet sind, dass sie jeweils mit wenigstens einer Elektrodenkontaktfläche (50) des ersten Elektrodenmoduls (10) korrespondieren.

13. Elektrodenstruktur nach Anspruch 12, wobei die Elektrodenkontaktflächen (50') und die Leiterbahnen (30') des wenigstens einen zweiten Elektrodenmoduls (20) so angeordnet sind, dass sie die Elektrodenkontaktflächen (50) des ersten Elektrodenmoduls (10) nicht abdecken.

14. Elektrodenstruktur nach einem der Ansprüche 12 oder 13, wobei die Elektrodenauflösung des ersten Elektrodenmoduls (10) verschieden von der Elektrodenauflösung des wenigstens einen zweiten Elektrodenmoduls (20) ist, wobei die Elektrodenauflösung als die Anzahl der Elektrodenkontaktflächen pro Flächeneinheit definiert ist.

15. Elektrodenstruktur nach einem der Ansprüche 12 bis 14, wobei das erste Substrat (71, 71') und/oder das zweite Substrat (72, 72') aus der Gruppe der synthetischen Polymere, vorzugsweise aus der Gruppe der Poly(organo)siloxane ausgewählt ist.

## Claims

1. Method for producing an electrode structure for a neural interface, wherein the method comprises:
- producing a first electrode module (10) and at least a second electrode module (20), wherein for the first electrode module (10) and for at least a second electrode module (20) respectively
- a number of electrode contact surfaces (50, 50') and a number of conductive paths (30, 30') are generated on a first substrate (71, 71'),
- a second substrate (72, 72') is applied onto the first substrate (71, 71') and the electrode contact surfaces (50, 50') and conductive paths (30, 30') generated thereon, and
- apertures are generated in the respective second substrate (72, 72') in the region of the electrode contact surfaces (50, 50'), and
- applying the at least one second electrode module (20) onto the first electrode module (10), wherein the first substrate (71') of the at least one electrode module (20) is arranged on the second substrate (72) of the first electrode module (10),
**characterized in that** apertures (60) are generated in the at least one second electrode module (20), which penetrate respectively the first substrate (71') and the second substrate (72') of the second electrode module (20), wherein the apertures (60) are arranged such that upon application of the second electrode module (20) onto the first electrode module (10) they respectively correspond to at least one electrode contact surface (50) of the first electrode module (10).

2. Method according to claim 1, wherein the electrode contact surfaces (50') and the conductive paths (30') of the at least one second electrode module (20) are generated such that they do not cover the electrode contact surfaces (50) of the first electrode module (10) upon application of the second electrode module (20) onto the first electrode module (10).

3. Method according to any one of the preceding claims, wherein the electrode resolution of the first electrode module (10) is selected so as to be different from the electrode resolution of at least one second electrode module (20), wherein the electrode resolution is defined as the number of electrode contact surfaces per unit area.

4. Method according to any one of the preceding claims, wherein prior to the application of the at least one second electrode module (20) onto the first electrode module (10) on the second substrate (72) of the first electrode module (10), a solvent is applied.

5. Method according to any one of the preceding claims, wherein in at least one of the electrode modules (10, 20), conduits are generated, which respectively have an inlet opening and at least one outlet opening for transport of a fluid.

6. Method according to any one of the preceding claims, wherein the first substrate (71, 71') and/or the second substrate (72, 72') are selected from the group of synthetic polymers, preferably, from the group of poly(organo)siloxanes.

7. Method according to any one of the preceding claims, wherein the first electrode module (10) and/or the at least one second electrode module (20) are designed computer-based.

8. Method according to any one of the preceding claims, wherein the electrode contact surfaces (50, 50') and conductive paths (30, 30') to be generated on the first substrate (71, 71') are generated by a laser cutting method on the first substrate (71, 71').

9. Method according to any one of the preceding claims, wherein in at least one of the electrode modules (10, 20) at least one optical fiber is integrated, which is lead to the outside at the second substrate (72, 72').

10. Method according to any one of the preceding claims, wherein prior to the application of the at least one second electrode module (20) onto the first electrode module (10), at least one second electrode module (20) is folded at least one time along a line (L1).

11. Method according to claim 10, wherein prior to the application of the at least one second electrode module (20) onto the first electrode module (10), at least one second electrode module (20) is folded one time along a first line (L1) such that the second substrate (72') is oriented outwards, and the at least one folded second electrode module (20) is bent along a second line (L2) and along a third line (L3) such that the resulting at least one second electrode module (20) represents in profile view an inverted T-section,

12. Electrode structure, in particular, for a neural interface, comprising a first electrode module (10) and at least one second electrode module (20) being produced separately from the first electrode module, which respectively comprise a first substrate (71, 71'), on which a number of electrode contact surfaces (50, 50') and a number of conductive paths (30, 30') are arranged, and a second substrate (72, 72'), which is arranged on the first substrate (71, 71') and the electrode contact surfaces (50, 50') and conductive paths (30, 30') arranged thereon, wherein the at least one second electrode module (20) is arranged on the first electrode module (10),
**characterized in that** apertures (60) are arranged in the at least one second electrode module (20), which respectively penetrate through the first substrate (71') and the second substrate (72') of the second electrode module (20), wherein the apertures (60) are arranged such that the respectively correspond to at least one electrode contact surface (50) of the first electrode module (10).

13. Electrode structure according to claim 12, wherein the electrode contact surfaces (50') and the conductive paths (30') of the at least one second electrode module (20) are arranged such that they do not cover the electrode contact surfaces (50) of the first electrode module (10).

14. Electrode structure according to any one of claims 12 or 13, wherein the electrode resolution of the first electrode module (10) is different from the electrode resolution of the at least one second electrode module (20), wherein the electrode resolution is defined as the number of electrode contact surfaces per unit area.

15. Electrode structure according to any one of claims 12 to 14, wherein the first substrate (71, 71') and/or the second substrate (72, 72') is selected from the group of synthetic polymers, preferably, from the group of poly(organo)siloxanes.

## Revendications

1. Procédé de fabrication d'une structure d'électrodes pour une interface neuronale, le procédé comprenant :
- Production d'un premier module d'électrodes (10) et d'au moins un deuxième module d'électrodes (20), dans lequel pour le premier module d'électrodes (10) et pour au moins un deuxième module d'électrodes (20), respectivement,
- un certain nombre de surfaces de contact d'électrodes (50, 50') et un certain nombre de pistes conductrices (30, 30') sont générés sur un premier substrat (71, 71'),
- un deuxième substrat (72, 72') est appliqué sur le premier substrat (71, 71') et sur les surfaces de contact d'électrodes (50, 50') et sur des pistes conductrices (30, 30') générée sur celui-ci, et
- des ouvertures sont générées dans le deuxième substrat respectif (72, 72') dans la zone des surfaces de contact d'électrode (50, 50'), et
- Application de l'au moins un deuxièmee module d'électrodes (20) sur le premier module d'électrodes (10), le premier substrat (71') de l'au moins un module d'électrodes (20) étant disposé sur le deuxième substrat (72) du premier module d'électrodes (10),
**caractérisé en ce que** des ouvertures (60) sont générées dans l'au moins un deuxième module d'électrode (20), qui pénètrent respectivement le premier substrat (71') et le deuxième substrat (72') du deuxième module d'électrodes (20), les ouvertures (60) étant disposées de telle sorte qu'elles correspondent respectivement, lors de l'application du deuxième module d'électrode (20) sur le premier module d'électrodes (10), à au moins une surface de contact d'électrode (50) du premier module d'électrodes (10).

2. Procédé selon la revendication 1, dans lequel les surfaces de contact d'électrode (50') et les pistes conductrices (30') de l'au moins un deuxième module d'électrodes (20) sont générées de telle sorte qu'elles ne recouvrent pas les surfaces de contact d'électrode (50) du premier module d'électrode (10) lors de l'application du deuxième module d'électrodes (20) sur le premier module d'électrodes (10).

3. Procédé selon l'une quelconque des revendications précédentes, dans lequel la résolution d'électrodes du premier module d'électrodes (10) est choisi de manière à être différente de la résolution d'électrodes de l'au moins un deuxième module d'électrodes (20), la résolution des électrodes étant définie comme étant le nombre d'électrodes des surfaces de contact par unité de surface.

4. Procédé selon l'une quelconque des revendications précédentes, dans lequel avant l'application de l'au moins un deuxième module d'électrodes (20) sur le premier module d'électrodes (10) sur le deuxième substrat (72) du premier module d'électrode (10), un solvant est appliqué.

5. Procédé selon l'une quelconque des revendications précédentes, dans lequel dans au moins un des modules d'électrodes (10, 20), des conduits sont générés, qui ont respectivement une ouverture d'entrée et au moins une ouverture de sortie pour le transport d'un fluide.

6. Procédé selon l'une quelconque des revendications précédentes, dans lequel le premier substrat (71, 71') et / ou le deuxième substrat (72, 72') sont choisis parmi le groupe des polymères synthétiques, de préférence dans le groupe des poly (organo) siloxanes.

7. Procédé selon l'une quelconque des revendications précédentes, dans lequel le premier module d'électrodes (10) et / ou l'au moins un deuxième module d'électrodes (20) sont conçus à base d'ordinateur.

8. Procédé selon l'une quelconque des revendications précédentes, dans lequel les surfaces de contact d'électrodes (50, 50') et les pistes conductrices (30, 30') à être générées sur le premier substrat (71, 71') sont générées sur le premier substrat (71, 71') par un procédé de découpage à laser.

9. Procédé selon l'une quelconque des revendications précédentes, dans lequel dans au moins l'un des modules d'électrodes (10, 20) au moins une fibre optique est intégrée, qui est conduite à l'extérieur au niveau du deuxième substrat (72, 72').

10. Procédé selon l'une quelconque des revendications précédentes, dans lequel avant l'application de l'au moins un deuxième module électrodes (20) sur le premier module d'électrodes (10), au moins un deuxième module d'électrodes (20) est plié au moins une fois le long d'une ligne (L1).

11. Procédé selon la revendication 10, dans lequel avant l'application de l'au moins un deuxième module d'électrodes (20) sur le premier module d'électrodes (10), au moins un deuxième module d'électrodes (20) est plié une fois le long d'une première ligne (L1) de sorte que le deuxième substrat (72') est orienté vers l'extérieur, et ledit au moins un deuxième module d'électrodes replié (20) est plié le long d'une deuxième ligne (L2) et le long d'une troisième ligne (L3) de sorte que le résultant au moins un deuxième module d'électrodes (20) représente en vue de profil une section en T inversé.

12. Structure d'électrodes, en particulier, pour une interface neuronale, comprenant un premier module d'électrodes (10) et au moins un deuxième module d'électrodes (20) étant produit séparément du premier module d'électrodes, qui comprennent respectivement un premier substrat (71, 71'), sur lequel un certain nombre de surfaces de contact d'électrodes (50, 50') et un certain nombre de pistes conductrices (30, 30') sont disposés, et un deuxième substrat (72, 72'), qui est disposé sur le premier substrat (71, 71') et les surfaces de contact d'électrodes (50, 50') et les pistes conductrices (30, 30') disposées sur celui-ci, dans lequel l'au moins un deuxième module d'électrodes (20) est disposé sur le premier module d'électrodes (10), **caractérisé en ce que** des ouvertures (60) sont disposés dans l'au moins un seocond module d'électrodes (20), qui pénètrent respectivement à travers le premier substrat (71') et le deuxième substrat (72') du deuxième module d'électrodes (20), les ouvertures (60) étant disposées de telle sorte qu'elles correspondent respectivement à au moins une surface de contact d'électrodes (50) du premier module d'électrode (10)

13. Structure d'électrodes selon la revendication 12, dans lequel les surfaces de contact d'électrode (50') et les pistes conductrices (30') de l'au moins un deuxième module d'électrode (20) sont disposées de telle sorte qu'elles ne recouvrent pas les surfaces de contact d'électrodes (50) du premier module d'électrodes (10).

14. Structure d'électrodes selon l'une quelconque des revendications 12 ou 13, dans lequel la résolution d'électrodes du premier module d'électrodes (10) est différente de la résolution d'électrodes de l'au moins un deuxième module électrodes (20), d la résolution d'électrodes étant définie comme le nombre d'électrodes des surfaces de contact par unité de surface.

15. Structure d'électrodes selon l'une quelconque des revendications 12 à 14, dans lequel le premier substrat (71, 71') et / ou le deuxième substrat (72, 72') sont choisi dans le groupe des polymères synthétiques, de préférence dans le groupe de poly (organo) siloxanes.
